# EUROPEAN PATENT SPECIFICATION

(11) **EP 2 977 029 B1**
(45) Date of publication and mention of the grant of the patent: **06.06.2018**
(21) Application number: 15175411.6
(22) Date of filing: 06.07.2015
(51) Int. Cl.: A61F 2/44

(54) **AUTOMATIC ELASTIC EXPANDABLE SPINAL IMPLANT**
AUTOMATISCHES ELASTISCHES EXPANDIERBARES WIRBELSÄULENIMPLANTAT
IMPLANT VERTÉBRAL EXTENSIBLE ÉLASTIQUE AUTOMATIQUE

(30) Priority: 22.07.2014 TW 103125170
(43) Date of publication of application: 27.01.2016
(73) Proprietor: OSSAWARE Biotech Co., Ltd., Changhua County 509 (TW)
(72) Inventor: CHEN, Kuei-Jung, 507 Xianxi Township, Changhua County, (TW)
(74) Representative: Viering, Jentschura & Partner mbB Patent- und Rechtsanwälte

(56) References cited:
- WO-A1-00/25706
- WO-A1-2010/105181
- WO-A1-2014/035835
- WO-A2-2012/007918
- US-A1- 2011 009 969
- US-A1- 2011 276 141
- US-A1- 2013 079 883

## Description

### BACKGROUND OF THE INVENTION

### 1. Field of the Invention

The present invention relates to an automatic elastic expandable spinal implant for a minimally invasive spine surgery, and more particularly to an automatic elastic expandable spinal implant which can expand or bounce or retract automatically.

### 2. Description of the Prior Art

These days, in order to solve the spinal degeneration due to old age or other external forces to cause discomfort, a common way adopts an intervertebral fusion operation or an artificial intervertebral disc implanted into vertebral. A minimally invasive surgery has the advantages of a small wound, a rapid speed of recovery, and a low risk of infection. Therefore, the minimally invasive techniques are the development trend of the surgery.

For example, Taiwan Patent No. I236368, M426388, I407938, M400307 and U.S. Patent No. 5888227 and U.S. Patent Early Publication No. 20130173003, 20080147193 disclose spinal implants. These implants have a fixed configuration or shape and are unable to expand or bounce or retract automatically. The size of the implants is big, so it is not suitable for a minimally invasive surgery.

In addition, U.S. Patent No. 8,241,358 and U.S. Patent Early Publication No. 2013/0079883 also disclose spinal implants. US 2011/0276141 A1 discloses an elastic expandable spinal implant according to the preamble of appended claim 1. These implants can be expanded or retracted by using an operating tool. However, it is necessary to compress or push the components or parts all the way to expand or retract the implant. The components or parts are unable to expand or bounce or fold automatically. This will prolong the time of surgery. Furthermore, the area to retain the vertebral is less, so the vertebral implant is unstable. Accordingly, the inventor of the present invention has devoted himself based on his many years of practical experiences to solve these problems.

### SUMMARY OF THE INVENTION

In view of this, the inventor enthusiastically develops and researches in order to invent an implant which can expand or bounce or retract automatically for convenient operation, shortening the time of operation, enhancing the stability of vertebra, and increasing the success rate of operation.

The primary object of the present invention is to provide an automatic elastic expandable spinal implant which comprises three or more than three members pivotally or hingedly connected together, enabling the implant to expand or bounce or retract automatically. The implant can be operated more conveniently.

A further object of the present invention is to provide an automatic elastic expandable spinal implant which can be collapsed to a smaller size so as to reduce the wound of the patient effectively.

In order to achieve the aforesaid objects, the automatic elastic expandable spinal implant comprises a main body and two link rods. The main body comprises a base portion and two side extension portions. The two side extension portions have distal ends, respectively. The two link rods are disposed inside the base portion and the two side extension portions of the main body. The two link rods have outer ends hingedly connected with the distal ends of the two side extension portions of the main body at a second pivot portion and a third pivot portion, respectively. The two link rods have inner ends, respectively. The inner ends of the two link rods are hingedly connected with each other at a first pivot portion. At least one of the main body and the two link rods is capable of elastic deformation. When the two link rods are pushed or pulled a predetermined distance from the base portion of the main body, the two link rods automatically expand away from the base portion of the main body. The operation of the present invention is more improved.

Compared to the existing technique, the present invention can expand or bounce or retract automatically, be operated conveniently, shorten the time of operation, enhance the stability of vertebra, and increase the success rate of operation.

### BRIEF DESCRIPTION OF THE DRAWINGS

FIG. 1 is a perspective view of the present invention;
FIG. 2 is a top view of the present invention;
FIG. 3 and FIG. 4 are enlarged top views showing the hinged portion of the present invention;
FIG. 5 is a side view of the present invention;
FIG. 6 is a schematic view of the present invention, showing that the two link rods are pushed outward beyond the predetermined distance to expand automatically;
FIG. 7, FIG. 8 and FIG. 9 are schematic views showing that the present invention is implanted into the vertebra during operation;
FIG. 10 is a schematic view of the present invention, showing that the two link rods are pushed inward beyond the predetermined distance to retract automatically;
FIG. 11 is a schematic view of the present invention, showing that the outer ends of the two link rods are provided with the lugs;
FIG. 12 and FIG. 13 are schematic views of the present invention, showing that the two link rods are provided with elastic members;
FIG. 14 and FIG. 15 are schematic views of the present invention, showing that a hinge member is provided between the two link rods;
FIG. 16 and FIG. 17 are partial enlarged views showing another hinge embodiment of the present invention;
FIG. 18 is a top view of the present invention, showing that the two link rods are formed with split grooves; and
FIG. 19 is a side view of the present invention, showing that the two link rods are formed with split grooves.

### DETAILED DESCRIPTION OF THE PREFERRED EMBODIMENTS

Embodiments of the present invention will now be described, by way of example only, with reference to the accompanying drawings.

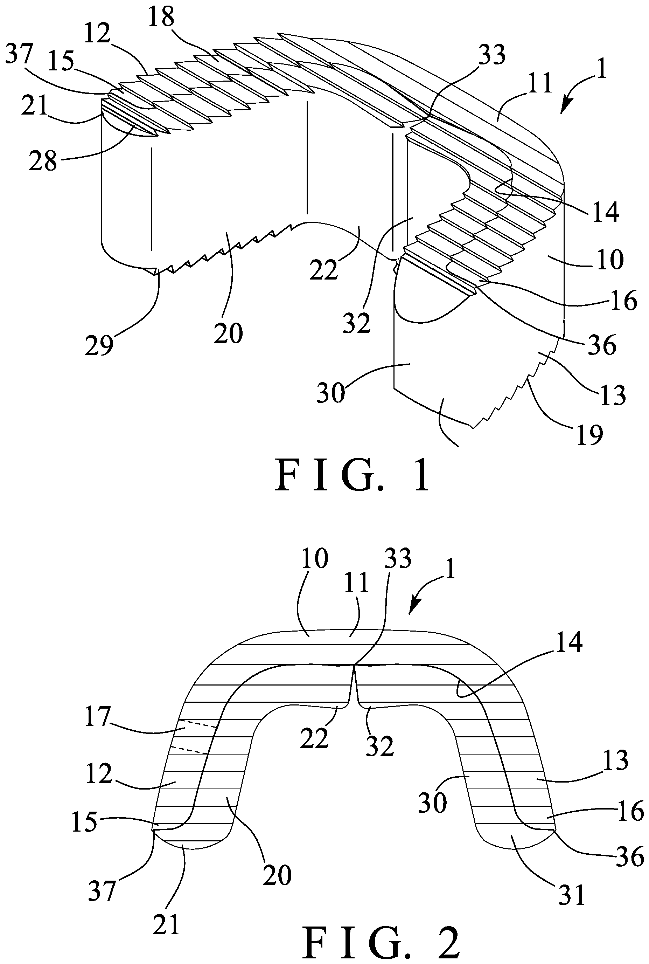

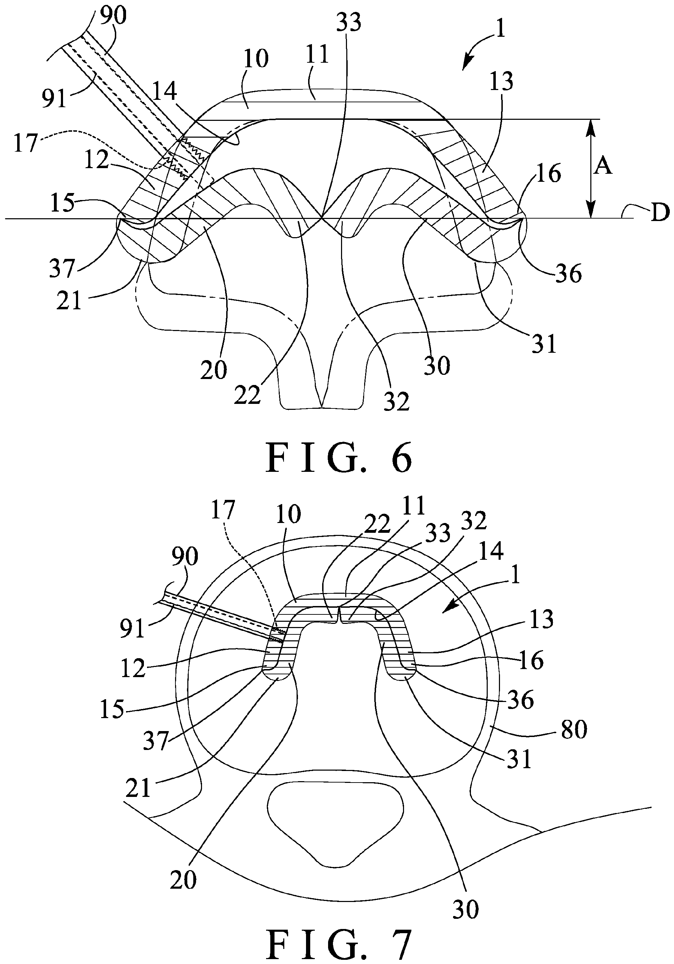

The present invention relates to an automatic elastic expandable spinal implant 1. As shown in FIG. 1 to FIG. 5, the implant 1 is provided for implantation for a damaged bone, particularly a vertebra 80. As shown in FIG. 7 to FIG. 9, the implant 1 is used for a minimally invasive spine surgery, so that the implant 1 can be used to support the damaged or compressed vertebra 80. The implant 1 comprises a main body 10 and two link rods 20, 30. The main body 10 generally has a curved shape, an arc shape, or a bow shape. The main body 10 comprises a base portion 11 and two side extension portions 12, 13 which are generally in an integral configuration to form a concave accommodation room 14 therein. The two side extension portions 12, 13 have outer ends or distal ends 15, 16, respectively.

The two link rods 20, 30 are disposed in the accommodation room 14 defined by the base portion 11 and the two side extension portions 12, 13 of the main body 10. The two link rods 20, 30 have outer ends 21, 31 pivotally or hingedly connected with the distal ends 15, 16 of the two side extension portions 12, 13 of the main body 10 to form a second pivot portion 36 and a third pivot portion 37, respectively. The two link rods 20, 30 have inner ends 22, 32 each generally in an L shape, an arc shape or other shape. The inner ends 22, 32 of the two link rods 20, 30 are pivotally or hingedly connected with each other to form a first pivot portion 33. At least one of the main body 10 and the two link rods 20, 30 is capable of elastic deformation.

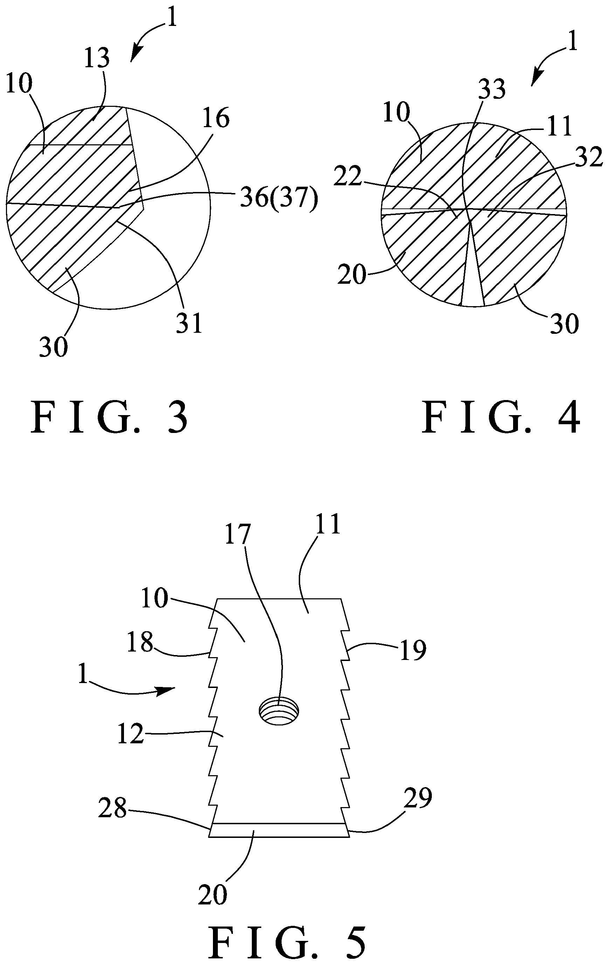

As shown in FIG. 1, FIG. 2 and FIG. 4, the inner ends 22, 32 of the two link rods 20, 30 are pivoted or hinged at the first pivot portion 33. When the two link rods 20, 30 are accommodated in the accommodation room 14 of the main body 10 (as shown in FIG. 1 and FIG. 2), the two link rods 20, 30 will press against or contact with the base portion 11 of the main body 10. The first pivot portion 33 for the two link rods 20, 30 to be pivotally or hingedly connected with each other is located at a hinged joint (as shown in FIG. 4) close to or contact with the base portion 11 of the main body 10. The second pivot portion 36 and the third pivot portion 37 for the outer ends 21, 31 of the two link rods 20, 30 to be pivotally or hingedly connected with the distal ends 15, 16 of the two side extension portions 12, 13 of the main body 10 are located at another two hinged joints (as shown in FIG. 3), enabling the main body 10 and the two inner ends 22, 32 of the two link rods 20, 30 of the main body 10 to be an integral configuration.

As shown in FIG. 2, FIG. 5 and FIG. 6, the main body 10 is provided with a threaded hole 17 defined in the base portion 11 or one of the two side extension portions 12, 13 for engagement or insertion of a spindle 91 of an operating tool 90, as shown in FIG. 6 to FIG. 8.

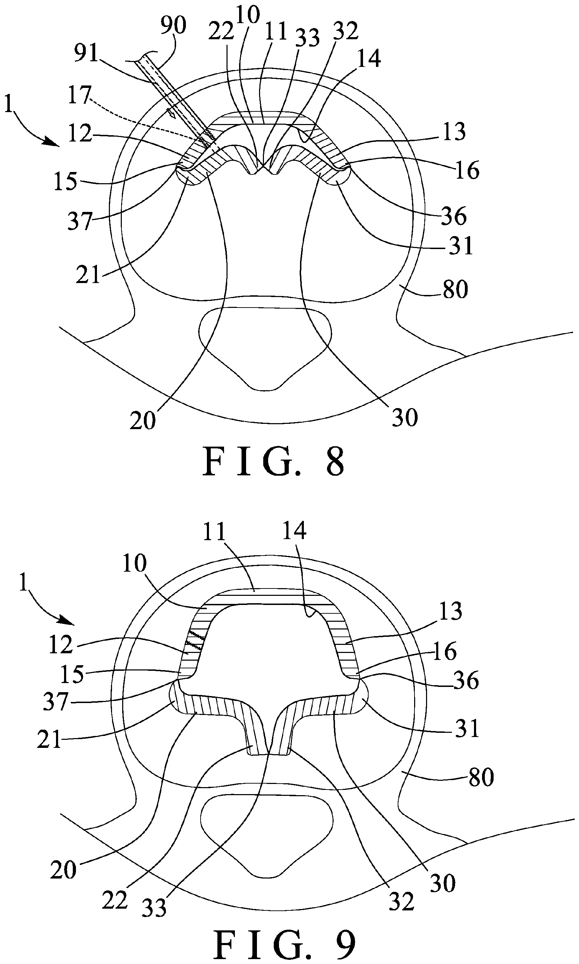

When in operation, as shown in FIG. 7 to FIG. 11, the implant 1 is implanted into the damaged bone, particularly the vertebra 80, by the user, the spindle 91 of the operating tool 90 is engaged with or inserted into the threaded hole 17 of the main body 10 to press or contact with one of the link rods 20, 30, such that the two link rods 20, 30 are pushed away from the main body 10. When the two link rods 20, 30 are pushed a predetermined distance A from the position close to the innermost side of the main body 10 or a folded position to the position of a dead point D, as shown in FIG. 6 and FIG. 8, the two link rods 20, 30 will automatically expand away from the main body 10 to the outmost open position or the working position as shown in FIG. 9.

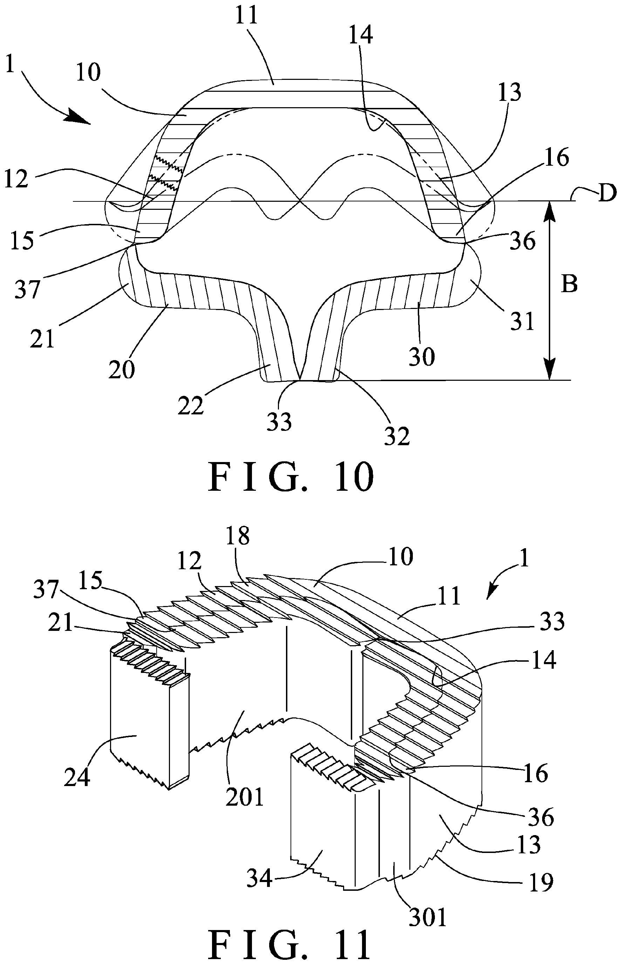

On the contrary, as shown in FIG. 10, when the two link rods 20, 30 are pushed to move a predetermined distance B from the outmost open position or the working position as shown in FIG. 9 toward the main body 10 to the position of the dead point D, the two link rods 20, 30 will automatically retract toward the main body 10 to the innermost position or the folded position as shown in FIG. 7. The dead point D of the predetermined distance A, B is defined at a position where a straight line is formed by the second pivot portion 36 and the third pivot portion 37 when the first pivot portion 33 traverse there.

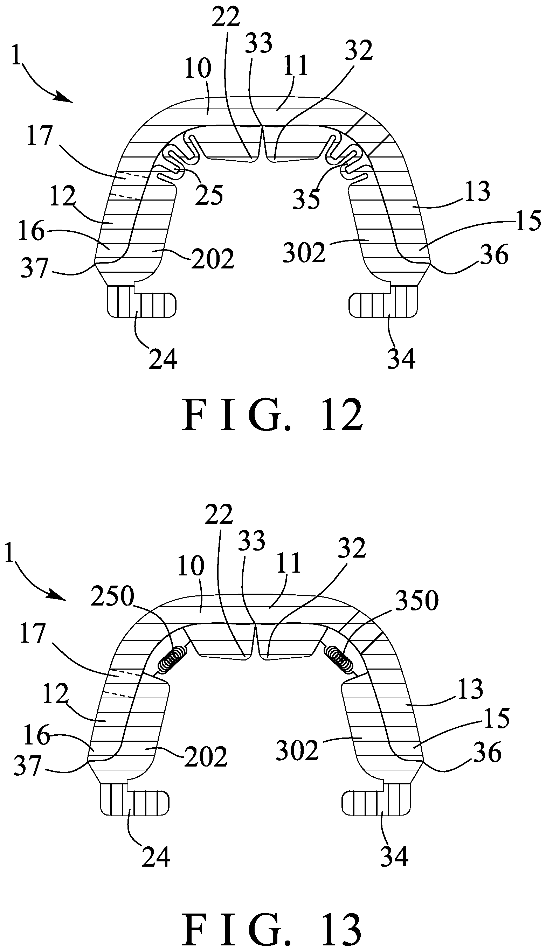

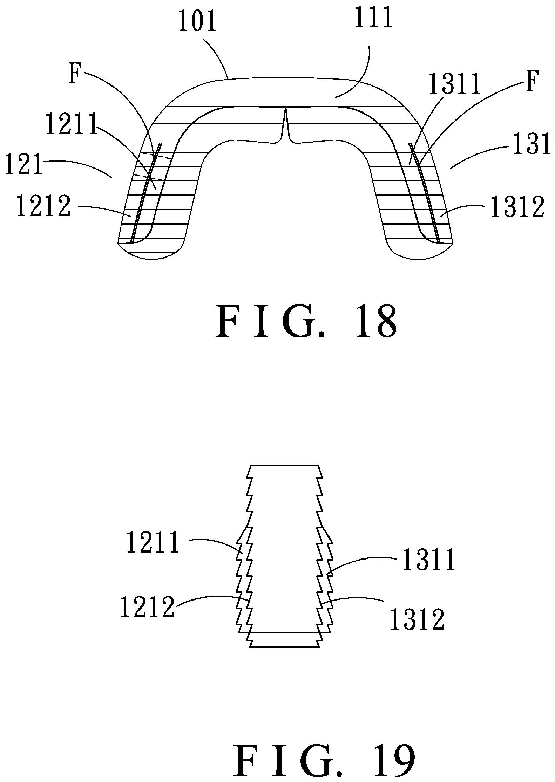

As shown in FIG. 11, the two link rods 201, 301 have lugs 24, 34 to increase the support area of the implant 1. As shown in FIG. 12, the two link rods 202, 302 have wavy elastic members 25, 35 to increase the flexibility of the two link rods 202, 302. Alternatively, as shown in FIG. 13, the two link rods 202, 302 have elastic members 250, 350 to increase the flexibility of the two link rods 202, 302. The wavy elastic members 25, 35 or the elastic members 250, 350 can be disposed on the main body 10. As shown in FIG. 18 and FIG. 19, the two side extension portions 121, 131 of the main body 101 is provided with split grooves F extending inward from the distal ends to approach the base portion 111, such that the side extension portions 121, 131 are divided into inner side extension portions 1211, 1311 and outer side extension portions 1212, 1312, respectively. Upper and lower surfaces of the inner side extension portions 1211, 1311 are higher than upper and lower surfaces of the outer side extension portions 1212, 1312, enabling the inner side extension portions 1211, 1311 to bring a reverse engagement for preventing the implant 1 from disengaging from the vertebra.

It is noted that when the two link rods 20, 30 are pushed the predetermined distance A from the position closest to the innermost side of the main body 10 or the folded position to the position of a dead point D (as shown in FIG. 6 and FIG. 8), the two link rods 20, 30 will automatically expand from the main body 10 to the outmost open position or the working position as shown in FIG. 9. It is not necessary to push the two link rods 20, 30 all the way from the position closest to the innermost side of the main body 10 or the folded position to the outmost open position or the working position as shown in FIG. 9. It is also not necessary to push the two link rods 20, 30 all the way from the outmost open position or the working position to the innermost side or the folded position as shown in FIG. 7. When the two link rods 20, 30 automatically expand away from the main body 10 to the outmost open position or the working position as shown in FIG. 9, the space between the main body 10 and the two link rods 20, 30 can be filled with autogenous bones or artificial bones or other stuff for performing a vertebral fusion operation.

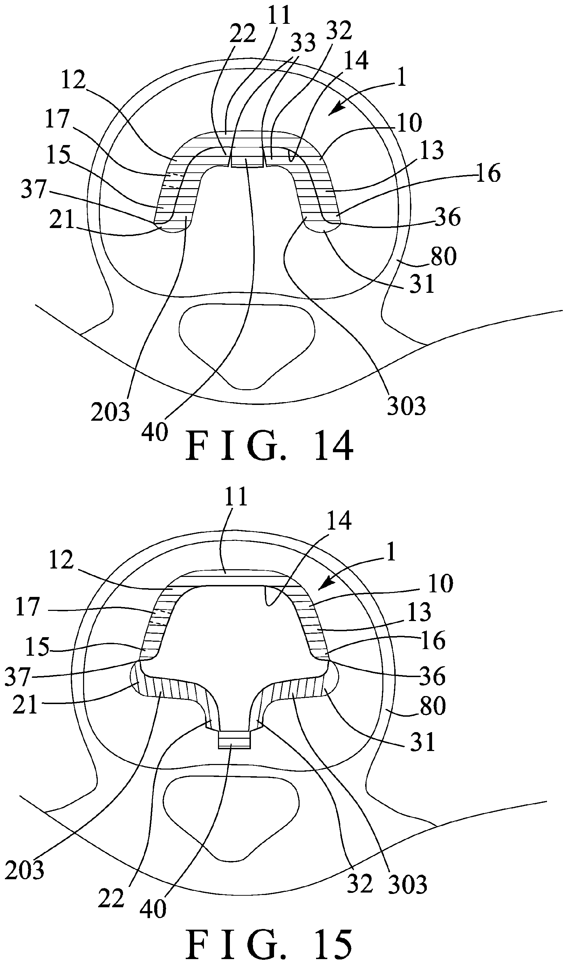

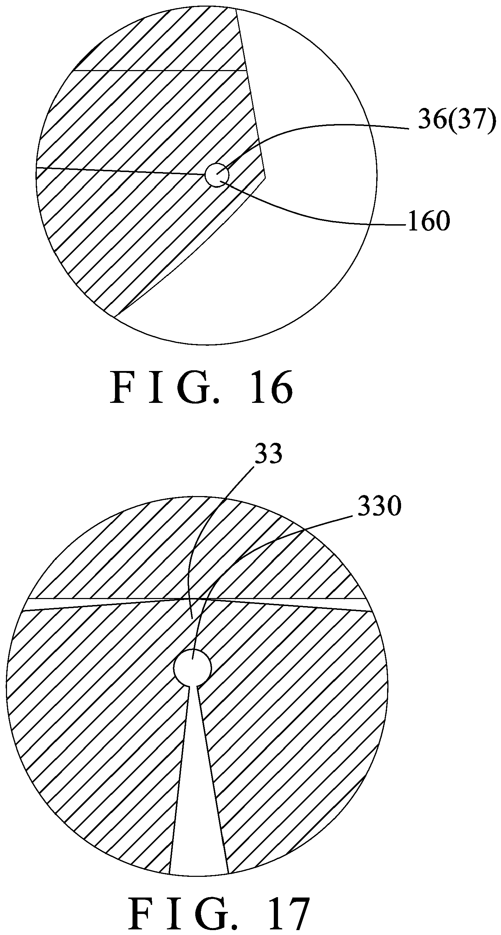

Furthermore, the outer surfaces of the main body 10 and the two link rods 20, 30 can be provided with serrated surfaces 18, 19, 28, 29 respectively to increase friction between the implant 1 and the bone or the vertebra 80. Alternatively, as shown in FIG. 14 and FIG. 15, the implant 1 may be provided with a hinge rod or a hinge member 40 which is pivotally or hingedly connected between the two link rods 203, 303 to increase the flexibility of the two link rods 203, 303. As shown in FIG. 16, each hinged joint of the second pivot portion 36 and the third pivot portion 37 may be formed with a buffering space 160 in a circle shape. As shown in FIG. 17, the hinged joint of the first pivot portion 33 may be formed with a buffering space 330 in a circle shape.

The appearances or shapes or configurations of the main body 10 and the two link rods 20, 30 can be various embodiments. Although particular embodiments of the present invention have been described in detail for purposes of illustration, various modifications and enhancements may be made without departing from the scope of the present invention. Accordingly, the present invention is not to be limited except as by the appended claims.

## Claims

1. An elastic expandable spinal implant, comprising:
a main body (10) comprising a base portion (11) and two side extension portions (12, 13), the two side extension portions (12, 13) having distal ends (15, 16) respectively; and
two link rods (20, 30) disposed inside the base portion (11) and the two side extension portions (12, 13) of the main body (10), the two link rods (20, 30) having outer ends (21, 31) hingedly connected with the distal ends (15, 16) of the two side extension portions (12, 13) of the main body (10) at a second pivot portion (36) and a third pivot portion (37) respectively, the two link rods (20, 30) having inner ends (22, 32) respectively, the inner ends (22, 32) of the two link rods (20, 30) being hingedly connected with each other at a first pivot portion (33), at least one of the main body (10) and the two link rods (20, 30) being capable of elastic deformation, **characterised in that** when the two link rods (20, 30) are pushed or pulled a predetermined distance (A) from the base portion (11) of the main body (10) to the position of a dead point (D), the two link rods (20, 30) expand away from the base portion (11) of the main body (10) due to the elastic deformability of the at least one of the main body and the two link rods.

2. The elastic expandable spinal implant as claimed in claim 1, wherein when the two link rods (20, 30) are pushed a predetermined distance (B) inward from a maximum open position toward the base portion (11) of the main body (10), the two link rods (20, 30) elastically retract toward the base portion (11) of the main body (10).

3. The elastic expandable spinal implant as claimed in claim 1, wherein the two link rods (20, 30) each have an L shape or an arc shape.

4. The elastic expandable spinal implant as claimed in claim 1, wherein the first pivot portion (33) for the two link rods (20, 30) to be hingedly connected with each other is located at a hinged joint close to the base portion (11) of the main body (10), enabling the two link rods (20, 30) to be an integral configuration.

5. The elastic expandable spinal implant as claimed in claim 1, wherein the second pivot portion (36) and the third pivot portion (37) for the two link rods (20, 30) to be hingedly connected with the two side extension portions (12, 13) of the main body (10) are located at two hinged joints close to outer sides thereof, enabling the two link rods (20, 30) and the main body (10) to be an integral configuration.

6. The elastic expandable spinal implant as claimed in claim 1, wherein the predetermined distance (A) is defined at a position where a straight line is formed by the second pivot portion (36) and the third pivot portion (37) when the first pivot portion (33) traverse there.

7. The elastic expandable spinal implant as claimed in claim 1, wherein the main body (10) is provided with a threaded hole (17) for engagement of an operating tool (90).

8. The elastic expandable spinal implant as claimed in claim 1, wherein outer surfaces of the main body (10) and the two link rods (20, 30) are provided with serrated surfaces (18, 19, 28, 29) respectively to increase friction.

9. The elastic expandable spinal implant as claimed in claim 1, wherein the two link rods (201, 301) have corresponding lugs (24, 34) to increase a support area.

10. The elastic expandable spinal implant as claimed in claim 1, wherein at least one of the main body (10) and the two link rods (202, 302) have wavy elastic members (25, 35) to increase flexibility of the two link rods (202, 302).

11. The elastic expandable spinal implant as claimed in claim 1, wherein at least one of the main body (10) and the two link rods (202, 302) have elastic members (250, 350) to increase flexibility of the two link rods (202, 302).

12. The elastic expandable spinal implant as claimed in claim 1, wherein a hinge member (40) is hingedly connected between the two link rods (203, 303) to increase flexibility of the two link rods (203, 303).

13. The elastic expandable spinal implant as claimed any one of claims 1 to 12, wherein the two side extension portions (121, 131) of the main body (101) are provided with split grooves (F) extending inward from the distal ends (15, 16) to approach the base portion (111), such that the side extension portions (121, 131) are divided into inner side extension portions (1211, 1311) and outer side extension portions (1212, 1312) respectively, and upper and lower surfaces of the inner side extension portions (1211, 1311) are higher than upper and lower surfaces of the outer side extension portions (1212, 1312), enabling the inner side extension portions (1211, 1311) to bring a reverse engagement.

## Patentansprüche

1. Ein elastisches, erweiterbares Wirbelsäulenimplantat, umfassend:
einen Grundkörper (10), der einen Basisanteil (11) und zwei seitliche Verlängerungsteile (12, 13) umfasst, wobei diese zwei seitliche Verlängerungsteile (12, 13) je ein distales Ende (15, 16) aufweisen; und
zwei Verbindungsstangen (20, 30), die inwendig im Basisanteil (11) mit den zwei seitlichen Verlängerungsteilen (12, 13) des Grundkörpers (10) vorgesehen sind; die zwei Verbindungsstangen (20, 30) äußere Endstücke (21, 31) aufweisen, die über ein Scharnier an den distalen Enden (15, 16) der zwei seitlichen Verlängerungsteile (12, 13) des Grundkörpers (10) an einem zweiten Drehzapfen (36) bzw. an einem dritten Drehzapfen (37) befestigt sind; die beiden Verbindungsstangen (20, 30) je innere Endstücke (22, 32) aufweisen, wobei diese inneren Endstücke (22, 32) der beiden Verbindungsstangen (20, 30) mit einem Scharnier aneinander an einem ersten Drehzapfen (33) befestigt sind; mindestens einer der Grundkörper (10) und die beiden Verbindungsstangen (20, 30) elastische verformbar sind, **dadurch gekennzeichnet, dass** beim Schieben oder Ziehen der beiden Verbindungsstangen (20, 30) um eine vorgegebene Distanz (A) vom Basisanteil (11) des Grundkörpers (10) in die Position eines toten Punkts (D) die beiden Verbindungsstangen (20, 30) vom Basisanteil (11) des Grundkörpers (10) wegen der elastischen Verformbarkeit des mindestens einen der Grundkörper und der zwei Verbindungsstangen weg bewegt werden.

2. Das elastische erweiterbare Wirbelsäulenimplantat nach Anspruch 1, wobei beim Schieben der zwei Verbindungsstangen (20, 30) um eine vorgegebene Distanz (B) von einer maximal offenen Position nach innen auf den Basisanteil (11) des Grundkörpers (10) zu die beiden Verbindungsstangen (20, 30) elastisch auf den Basisanteil (11) des Grundkörper (10) zu eingezogen werden.

3. Das elastische erweiterbare Wirbelsäulenimplantat nach Anspruch 1, wobei die zwei Verbindungsstangen (20, 30) beide je eine L-Form oder eine gebogene Form aufweisen.

4. Das elastische erweiterbare Wirbelsäulenimplantat nach Anspruch 1, wobei der erste Drehzapfen (33) für die zwei Verbindungsstangen (20, 30), um diese über ein Scharnier aneinander zu befestigen, an einem Scharniergelenk nahe zum Basisanteil (11) des Grundkörpers (10) angeordnet sind, um die beiden Verbindungsstangen (20, 30) integral miteinander zu konfigurieren.

5. Das elastische erweiterbare Wirbelsäulenimplantat nach Anspruch 1, wobei der zweite Drehzapfen (36) und der dritte Drehzapfen (37) für die beiden Verbindungsstangen (20, 30), die über ein Scharnier mit den beiden seitlichen Verlängerungsteilen (12, 13) des Grundkörpers (10) befestigt werden müssen, an zwei Scharniergelenken nahe zu deren Außenseiten angeordnet sind, um die beiden Verbindungsstangen (20, 30) und den Grundkörper (10) integral miteinander zu konfigurieren.

6. Das elastische erweiterbare Wirbelsäulenimplantat nach Anspruch 1, wobei die vorgegebene Distanz (A) in einer Position definiert wird, in der mit dem zweiten Drehzapfen (36) und mit dem dritten Drehzapfen (37) eine gerade Linie gebildet wird, wenn der erste Drehzapfen (33) quer über diese verläuft.

7. Das elastische erweiterbare Wirbelsäulenimplantat nach Anspruch 1, wobei der Grundkörper (10) ein Gewindeloch (17) zum Einrasten eines Betätigungswerkzeugs (90) aufweist.

8. Das elastische erweiterbare Wirbelsäulenimplantat nach Anspruch 1, wobei die Außenflächen des Grundkörpers (10) und die beiden Verbindungsstangen (20, 30) je gezackte Oberflächen (18, 19, 28, 29) aufweisen, um die Reibung zu erhöhen.

9. Das elastische erweiterbare Wirbelsäulenimplantat nach Anspruch 1, wobei die beiden Verbindungsstangen (201, 301) entsprechende Ösen (24, 34) aufweisen, um eine Auflagefläche zu vergrößern.

10. Das elastische erweiterbare Wirbelsäulenimplantat nach Anspruch 1, wobei der mindestens eine der Grundkörper (10) und die beiden Verbindungsstangen (202, 302) gewellte elastische Glieder (25, 35) aufweisen, um die Flexibilität der beiden Verbindungsstangen (202, 302) zu vergrößern.

11. Das elastische erweiterbare Wirbelsäulenimplantat nach Anspruch 1, wobei der mindestens eine der Grundkörper (10) und die beiden Verbindungsstangen (202, 302) elastische Glieder (250, 350) aufweisen, um die Flexibilität der beiden Verbindungsstangen (202, 302) zu erhöhen.

12. Das elastische erweiterbare Wirbelsäulenimplantat nach Anspruch 1, wobei ein Scharnier (40) zwischen den beiden Verbindungsstangen (203, 303) befestigt ist, um die Flexibilität der beiden Verbindungsstangen (203, 303) zu erhöhen.

13. Das elastische erweiterbare Wirbelsäulenimplantat nach einem der Ansprüche 1 bis 12, wobei die zwei seitlichen Verlängerungsteile (121, 131) des Grundkörpers (101) geteilte Nuten (F) aufweisen, die sich von den distalen Enden (15, 16) nach innen auf den Basisanteil (111) zu erstrecken, so dass die seitlichen Verlängerungsteile (121, 131) in innere seitliche Verlängerungsteile (1211, 1311) bzw. in äußere seitliche Verlängerungsteile (1212, 1312) aufgeteilt sind, wobei die oberen und unteren Flächen der inneren seitlichen Verlängerungsteile (1211, 1311) höher als die oberen und unteren Oberflächen der äußeren seitlichen Verlängerungsteile (1212, 1312) sind, so dass die inneren seitlichen Verlängerungsteile (1211, 1311) in einen umgekehrten Eingriff gebracht werden können.

## Revendications

1. Implant vertébral extensible élastique, **caractérisé par le fait qu'**il comprend :
un corps principal (10) comprenant une partie de base (11) et deux partie d'extension latérales (12, 13), les deux partie d'extension latérales (12, 13) comportant des extrémités distales (15, 16) respectivement ; et
deux bras articulés (20, 30) placés à l'intérieur de la partie de base (11) et des deux partie d'extension latérales (12, 13) du corps principal (10), les deux bras articulés (20, 30) comportant des extrémités extérieures (21, 31) reliées de manière articulée aux extrémités distales (15, 16) des deux parties d'extension latérales (12, 13) du corps principal (10) d'une seconde partie formant pivot (36) et d'une troisième partie formant pivot (37) respectivement, les deux bras articulés (20, 30) comportant des extrémités intérieures (22, 32) respectivement, les extrémités intérieures (22, 32) des deux bras articulés (20, 30) étant reliées de manière articulée l'une à l'autre à une première partie formant pivot (33), au moins l'un parmi le corps principal (10) et les deux bras articulés (20, 30) étant capable de déformation élastique, **caractérisé en ce que** lorsque les deux bras articulés (20, 30) sont poussés ou tirés sur une distance prédéterminée (A) depuis la partie de base (11) du corps principal (10) vers la position d'un point mort (D), les deux bras articulés (20, 30) s'étendent en s'éloignant depuis la partie de base (11) du corps principal (10) du fait de la capacité de déformation élastique d'au moins l'un parmi le corps principal et les deux bras articulés.

2. Implant vertébral extensible élastique selon la revendication 1, **caractérisé par le fait que** lorsque les deux bras articulés (20, 30) sont poussés sur une distance prédéterminée (B) vers l'intérieur depuis une position ouverte maximale vers la partie de base (11) du corps principal (10), les deux bras articulés (20, 30) se rétractent de manière élastique vers la partie de base (11) du corps principal (10).

3. Implant vertébral extensible élastique selon la revendication 1, **caractérisé par le fait que** les deux bras articulés (20, 30) présentent chacun une forme en L ou une forme en arc.

4. Implant vertébral extensible élastique selon la revendication 1, **caractérisé par le fait que** la première partie formant pivot (33) pour relier de manière articulée les deux bras articulés (20, 30) l'un à l'autre est située à un joint articulé proche de la partie de base (11) du corps principal (10), permettant aux deux bras articulés (20, 30) d'être une configuration intégrale.

5. Implant vertébral extensible élastique selon la revendication 1, **caractérisé par le fait que** la seconde partie formant pivot (36) et la troisième partie formant pivot (37) pour relier les deux bras articulés (20, 30) de manière articulée aux deux partie d'extension latérales (12, 13) du corps principal (10) sont situées à deux joints articulés proches de côtés extérieurs associés, permettant aux deux bras articulés (20, 30) et au corps principal (10) d'être une configuration intégrale.

6. Implant vertébral extensible élastique selon la revendication 1, **caractérisé par le fait que** la distance prédéterminée (A) est définie à une position où une ligne droite est formée par la seconde partie formant pivot (36) et la troisième partie formant pivot (37) lorsque la première partie formant pivot (33) traverse là.

7. Implant vertébral extensible élastique selon la revendication 1, **caractérisé par le fait que** le corps principal (10) est muni d'un trou fileté (17) pour l'engagement d'un outil de travail (90).

8. Implant vertébral extensible élastique selon la revendication 1, **caractérisé par le fait que** les surfaces extérieures du corps principal (10) et des deux bras articulés (20, 30) sont munies de surfaces dentelées (18, 19, 28, 29) respectivement pour augmenter la friction.

9. Implant vertébral extensible élastique selon la revendication 1, **caractérisé par le fait que** les deux bras articulés (201, 301) comportent des pattes correspondantes (24, 34) pour augmenter une zone de support.

10. Implant vertébral extensible élastique selon la revendication 1, **caractérisé par le fait qu'**au moins l'un parmi le corps principal (10) et les deux bras articulés (202, 302) comportent des éléments élastiques ondulés (25, 35) pour augmenter la flexibilité des deux bras articulés (202, 302).

11. Implant vertébral extensible élastique selon la revendication 1, **caractérisé par le fait qu'**au moins l'un parmi le corps principal (10) et les deux bras articulés (202, 302) comportent des éléments élastiques (250, 350) pour augmenter la flexibilité des deux bras articulés (202, 302).

12. Implant vertébral extensible élastique selon la revendication 1, **caractérisé par le fait qu'**un élément d'articulation (40) est raccordé de manière articulée entre les deux bras articulés (203, 303) pour augmenter la flexibilité des deux bras articulés (203, 303).

13. Implant vertébral extensible élastique selon l'une quelconque des revendications 1 à 12, **caractérisé par le fait que** les deux parties d'extension latérales (121, 131) du corps principal (101) sont munies de rainures en deux parties (F) s'étendant vers l'intérieur depuis les extrémités distales (15, 16) pour approcher la partie de base (111), de sorte que les partie d'extension latérales (121, 131) sont divisées entre des partie d'extension latérales internes (1211, 1311) et des partie d'extension latérales externes (1212, 1312) respectivement, et les surfaces hautes et basses des partie d'extension latérales internes (1211, 1311) sont plus hautes que les surfaces hautes et basses des partie d'extension latérales externes (1212, 1312), permettant aux parties d'extension latérales internes (1211, 1311) de provoquer un engagement inversé.
